# FASCICULE DE BREVET EUROPEEN

(11) **EP 3 145 480 B1**
(45) Date de publication et mention de la délivrance du brevet: **15.07.2020**
(21) Numéro de dépôt: 15732763.6
(22) Date de dépôt: 13.05.2015
(51) Int. Cl.: A61K 8/34, A61K 8/64, A61Q 19/02, A61Q 19/08, A61K 8/92

(54) **PREPARATIONS COSMETIQUES ET/OU DERMATOLOGIQUES EXTEMPORANEES**
BEDARFSMÄSSIGE KOSMETISCHE UND/ODER DERMATOLOGISCHE ZUSAMMENSETZUNGEN
EXTEMPORANEOUS COSMETIC AND/OR DERMATOLOGIC COMPOSITIONS

(30) Priorité: 13.05.2014 FR 1401067
(43) Date de publication de la demande: 29.03.2017
(73) Titulaire: Universkin, 06000 Nice (FR)
(72) Inventeur: ARAMPATZIS, Charalampos, 06000 Nice (FR); MASSON Claudine, 13006 Marseille (FR)
(74) Mandataire: Célanie, Christian
(86) Numéro de dépôt international: PCT/FR2015/000097
(87) Numéro de publication internationale: WO 2015/173481

(56) Documents cités:
- EP-A1- 1 027 878
- FR-A1- 2 906 463
- FR-A1- 2 913 602
- DATABASE GNPD [Online] MINTEL; mars 2007 (2007-03), "Bronze Camouflage Age Defying Advanced Quadruple Bronzer", XP002735395, Database accession no. 677774
- DATABASE GNPD [Online] MINTEL; septembre 2007 (2007-09), "Age-Defying Cream", XP002735396, Database accession no. 781183

## Description

Le secteur technique de la présente invention est celui de la cosmétique et de la dermatologie et plus particulièrement celui des compositions cosmétiques et/ou dermatologiques préparées de manière extemporanée. Ces compositions peuvent notamment participer à l'amélioration esthétique générale de la peau du corps ou du visage.

Le dermatologue, le médecin esthétique et le pharmacien sont confrontés à des pathologies et problématiques très diverses dans le domaine cutané. On peut citer notamment l'acné, les allergies (eczéma, urticaire), les infections bactériennes, virales et parasitaires, le psoriasis, la rosacée, les vergetures, le vitiligo, la chute des cheveux et la séborrhée, les problèmes de pigmentation, les rides et ridules, l'amélioration de la texture cutanée, la régénération faciale, l'utilisation de plus en plus fréquente de techniques dermatologiques invasives comme les peelings, les lasers, etc.

Ces dernières années, des avancées significatives sont intervenues dans l'étude de la peau et ont permis le développement important de technologies d'évaluation et de réparation de la peau.

Malgré ces remarquables avancées, les possibilités de traitement, aussi bien en dermatologie qu'en cosmétique, sont limitées à l'utilisation de produits développés et formulés pour une application spécifique et sont mis sur le marché par des sociétés de dermatologie et de cosmétique de renom.

Quelques préparations peuvent être prescrites par les dermatologues et fabriqués extemporanément par des pharmaciens.

Cependant, l'ensemble de l'offre de soin et de traitement actuelle ne permet pas d'adapter un traitement personnalisé adaptant les différents principes actifs, les différentes concentrations de ces principes actifs et la synergie de traitements successifs d'une durée limitée.

Une tendance forte se dessine en cosmétique et dermatologie, celle du soin sur mesure.

Dans le brevet EP-2343692, on décrit un appareil pour formuler des compositions cosmétiques sur mesure. Cependant, on ne peut utiliser cet appareil que pour mélanger et distribuer des compositions purement cosmétiques telles qu'un vernis à ongle, un mascara, etc. Aucun diagnostic de la peau n'est donc nécessaire et l'appareil fonctionne de façon automatisée. De plus, les formulations préparées selon le brevet EP-2343692 nécessitent un lourd appareillage. Enfin, aucun exemple de composition n'est détaillé.

Le diagnostic préalable à la formulation, lié aux nouvelles technologies d'évaluation, est l'une des voies qu'on met en place pour offrir des formules adaptées à la peau d'un patient et suivant la pathologie dont il souffre. La formulation cosmétique ou dermatologique doit ainsi pouvoir être réalisée strictement pour un besoin ou des besoins particuliers de la peau.

Dans la demande WO-0191600, on décrit un processus de réalisation de compositions sur mesure, à l'aide d'un diagnostique préalable de la peau d'un patient, d'un appareil pour le mélange de produits choisis et la fourniture de ce mélange. Cependant, les moyens de réalisation technique des compositions cosmétiques ne sont pas expliqués, comme par exemple la nature chimique des composants.

Dans le brevet FR-2902995, on décrit une composition cosmétique ou dermatologique sous la forme d'une émulsion. L'émulsion est réalisée extemporanément en mélangeant une phase pulvérulente composée d'une huile et d'un solide poreux avec une phase hydrophile généralement aqueuse. Le problème de cette composition est qu'elle ne peut contenir qu'un nombre de principes actifs limités. En effet, le principe actif doit soit être solubilisé dans la phase hydrophile, ce qui limite son conditionnement en raison du risque de dégradation et développement bactérien, soit être intégré à la phase pulvérulente, ce qui peut provoquer des problèmes de stabilité, d'homogénéisation ou de formulation de cette phase pulvérulente. De plus, on doit adapter la phase pulvérulente et la phase hydrophile afin de réaliser l'émulsion de manière extemporanée, ce qui limite le nombre de principes actifs utilisables. Le brevet EP 1 027 878 A1 décrit des compositions dermatologiques/cosmétiques dans lesquelles un principe actif acide, dont l'acide ascorbique est solubilisé de manière extemporanée dans une base de solubilisation. Enfin, la composition cosmétique ou dermatologique selon le brevet FR-2902995 ne permet pas d'ajuster extemporanément la quantité ou la nature des principes actifs, en particulier lorsqu'on doit intégrer ces principes actifs dans le processus de fabrication de la phase pulvérulente.

Une composition cosmétique dite fraîche est apparue, consistant à mélanger des principes actifs à la dernière minute pour préserver leur utilisation sur la peau, et cela grâce à l'amélioration de procédés de conservation (produits déshydratés) tels que les packagings à double compartiment.

Une formule toute faite ne peut répondre de manière adaptée aux besoins évolutifs de la peau. Les compositions actuelles, qui contiennent un mélange d'actifs préalablement établi et impossible à modifier, ne permettent aucune souplesse d'utilisation.

Il n'existe toujours pas à l'heure actuelle de composition cosmétique sur mesure, préparée extemporanément, permettant d'intégrer des principes actifs d'une manière à la fois flexible qualitativement (nature du problème de peau), quantitative (degré de gravité du problème de peau), et stable (stabilité de la composition cosmétique, en particulier une émulsion et conservation du principe actif).

La présente invention vise à résoudre ces problématiques en proposant un produit préparé de manière extemporanée comprenant d'une part une base universelle et d'autre part un ou des principes actifs choisis grâce à un diagnostic personnalisé de la peau du patient.

L'invention a donc pour objet un produit extemporané pour des soins cosmétiques et/ou dermatologiques comprenant au moins :
- un ou plusieurs principes actifs acceptables dans le domaine des soins cutanés cosmétiques et/ou dermatologiques, sous forme pulvérulente ou liquide, choisis grâce à un diagnostic personnalisé de la peau du patient d'une part ;
- et une base universelle d'autre part, comprenant :
   - au moins une huile présentant une teneur massique en acide α-linolénique supérieure à 15% et un rapport massique oméga-6 sur oméga-3 inférieur ou égal à 5,
   - de l'eau,
   - de la glycérine,
   - un ou plusieurs émulsifiants,
   - au moins un autre alcool que la glycérine, acceptable dans les compositions cosmétiques et/ou dermatologiques,
   - au moins un peptide biomimétique de formule générale A-X-Gly-His-Lys-Y dans laquelle :
      - A représente un atome d'hydrogène ou un radical d'un acide monocarboxylique de formule générale R-COOH dans laquelle R représente un radical aliphatique comprenant un à onze atomes de carbone,
      - X représente une liaison simple, la lysine, l'arginine ou l'ornithine,
      - et Y représente un groupe -OH ou NH2,
ladite base universelle étant apte lors du mélange à renforcer, potentialiser, à compléter l'effet dudit ou desdits principes actifs et à solubiliser ledit ou lesdits principes actifs.

Le pourcentage en masse du principe actif peut varier suivant un pourcentage allant de 0,25 à 30%. Il va de soi que le pourcentage d'un actif bien précis dépend de l'efficacité du traitement défini. Ce pourcentage correspond à un dosage dont l'efficacité a été objectivée scientifiquement, et, de plus, qui est rigoureusement défini dans le cadre de contraintes réglementaire et toxicologiques.

Selon une caractéristique de l'invention, l'huile est présente dans la base universelle suivant une concentration massique comprise entre 5 et 25%, et de préférence entre 7,5 et 20%%.

Selon une autre caractéristique de l'invention, l'huile est choisie parmi les huiles suivantes : l'huile de cameline, connue aussi sous la dénomination huile de pépins de cameline, l'huile de graines de citrouille, l'huile de chanvre, huile de pépin de cassis l'huile de lin, huile d'argan, huile d'olive, huile d'onagre, huile de bourrache et l'huile de pérille.

Selon encore une autre caractéristique de l'invention, la glycérine est présente dans la base universelle suivant une concentration massique comprise entre 1 et 20%, et de préférence entre 1 et 10%.

Selon encore une autre caractéristique de l'invention, la base universelle comprend au moins un des émulsifiants dont le nom INCI UE est le suivant : sodium acrylate/sodium acryloyldimethyl taurate copolymer, polysorbate 80, xantham gum.

Selon encore une autre caractéristique de l'invention, la base universelle comprend au moins un ou plusieurs émulsifiants suivant une concentration massique totale comprise entre 1 et 5%.

Selon encore une autre caractéristique de l'invention, l'autre alcool est présent dans la base universelle suivant une concentration massique comprise entre 10 et 30%, et de préférence entre 15 et 25%.

Selon encore une autre caractéristique de l'invention, l'autre alcool de la base universelle est choisi dans le groupe constitué par l'éthanol, le propanol, le propanediol, le propan-2-ol, le propylène glycol, le méthylène glycol, ou le triméthylène glycol.

Selon encore une autre caractéristique de l'invention, le peptide biomimétique est présent dans la base universelle selon une concentration variant de 1 à 20 ppm, préférentiellement de 3 à 10 ppm.

Selon encore une autre caractéristique de l'invention, la base universelle comprend au moins un peptide biomimétique de formule H-Gly-His-Lys-OH.

Selon encore une autre caractéristique de l'invention, la base universelle comprend en outre de l'acide hyaluronique, préférentiellement suivant une concentration massique inférieure à 5%.

Selon encore une autre caractéristique de l'invention, un ou plusieurs principes actifs sont choisis dans le groupe constitué par l'acide férulique, la phlorétine, la SOD ou Super Oxyde Dismutase, le rétinol et/ou autres rétinoïdes, le niacinamide ou Vitamine B3, le DMAE ou diméthyle aminoéthanol, le resvératrol, les isofalvones riches en génistéine et daidzéine, l'acide phytique, l'acide kojique, l'acide glycolique, l'arbutine, l'acide azélaïque, la N-acétyl cystéine, la tyrosine, l'acide alpha-lipoïque, la vitamine C, la vitamine B5, les ceramides, l'acide Hyaluronique, les sels de zinc et de magnésium, l'acide salicylique, extraits végétaux riches en rutine, l'aloe vera, les madécassosides, la caféine, le silicium organique, la L-Carnitine, les lactopeptides et/ou bacteriocines, l'urée, l'acide lactique, l'acide mandélique, l'acide ursolique, le resorcinol, l'hydroquinone et ses dérivés, et /ou autres extraits végétaux riches en flavonoïdes qui peuvent avoir un effet bénéfique sur la peau.

Selon encore une autre caractéristique de l'invention, un ou plusieurs principes actifs sont choisis parmi les familles du groupe constitué par les flavonoïdes, les chalcones, les sels de zinc, de calcium et de magnésium de l'acide gluconique, les vitamines et les acides alpha et bêta-hydroxylés.

Un autre objet de l'invention concerne un kit de préparation extemporanée d'une composition cosmétique et/ou dermatologique mettant en œuvre un produit extemporané, caractérisé en ce qu'il est constitué d'un premier conditionnement étanche contenant la base universelle, et un second conditionnement étanche contenant au moins un principe actif, les deux conditionnements étant aptes à être solidarisés de façon réversible et comprenant un moyen permettant de réunir leur contenu par une simple manipulation ou pression.

Un tout premier avantage de l'invention réside dans la possibilité de préparer de très nombreuses formulations cosmétiques et/ou dermatologiques de manière extemporanée.

Un autre avantage de l'invention réside dans la stabilité dans le temps de la base universelle d'une part et des principes actifs d'autre part.

Un autre avantage encore de l'invention réside dans la simplicité de préparation de compositions cosmétiques et/ou dermatologiques de manière extemporanée.

Un autre avantage encore de l'invention réside dans l'ajustement de la nature et de la quantité de chaque principe actif selon respectivement le type d'affection ou de besoin physiologique de la peau et l'importance de cette affection ou de ce besoin.

Un autre avantage encore de l'invention réside dans la possibilité d'obtenir simplement des préparations extemporanées, c'est-à-dire préparées sur mesure par un pharmacien, un médecin esthétique, chirurgien plasticien ou un dermatologue, éventuellement d'après une recommandation et/ou ordonnance prescrite par un médecin, pour le traitement ou le soin cutané d'un patient donné, notamment d'une pathologie (ex. : l'acné, vergetures) et/ou d'un besoin (ex. : relâchement cutané, rides et ridules, cellulite), et pour des propriétés thérapeutiques (ex. : la dépigmentation).

Un autre avantage encore de l'invention réside dans la sélection par le pharmacien, le médecin ou le dermatologue du traitement de la peau en fonction du dysfonctionnement cutané réel.

Un autre avantage encore de l'invention réside dans le large spectre d'affection cutanées traitées ou de besoins cutanés comblés grâce à la présence, dans les compositions cutanées préparées de manière extemporanée, d'une part de peptides biomimétiques ayant une action biologique et d'autre part de principes actifs traditionnels ayant une action dynamique.

D'autres caractéristiques, détails et avantages de l'invention seront mieux compris à la lecture du complément de description donné à titre d'exemple en relation avec des dessins sur lesquels :
- la figure 1 représente un flacon contenant la base universelle vissé avec un bouchon contenant un principe actif, et
- la figure 2 représente un flacon contenant la base universelle vissé avec un bouchon déversant un principe actif dans le flacon.

L'invention est destinée à l'utilisation du produit extemporané par les officines des pharmaciens, des dermatologues, des médecins généralistes ou esthétiques pour l'élaboration de compositions cosmétiques et/ou dermatologiques préparées de manière extemporanée suivant le besoin dermatologique et/ou cosmétique du patient.

Les demandeurs ont ainsi découvert, après de longues et patientes recherchés, un produit extemporané comprenant d'une part une base universelle et d'autre part un ou des principes actifs. Ladite base comprend un choix précis d'un alcool, d'une huile et de l'eau afin de solubiliser une très grande variété de principes actifs solides ou liquides.

Par produit extemporané, on entend un produit en deux parties, comprenant d'une part la base universelle et d'autre part un ou des principes actifs purs et/ou dilués, ce produit extemporané étant destiné à la préparation d'une composition cosmétique et/ou dermatologique de manière extemporanée.

Par base universelle, on entend une composition capable de solubiliser un grand nombre de principe actifs solides ou liquides et comprenant au moins une huile riche en acides gras essentiels polyinsaturés et plus particulièrement en oméga-3, de l'eau, de la glycérine, au moins un autre alcool que la glycérine acceptable en cosmétique et/ou en dermatologie, un ou plusieurs agents émulsifiants, et au moins un peptide biomimétique. La base universelle présente en outre une grande stabilité dans le temps, ce qui facilite sa conservation en vue de préparer une composition de manière extemporanée. La base universelle peut notamment être une émulsion.

Cette base universelle est capable de solubiliser les différents principes actifs, solides ou liquides, en restant stable au moins pendant 30 jours, ce qui représente deux fois la durée prescrite d'utilisation de la composition préparée de manière extemporanée qui est donc de 15 à 30 jours.

Cette base présente l'avantage important de satisfaire tous les besoins des praticiens dans le choix du traitement préconisé en définissant le principe actif lui-même et la dose à incorporer. Il n'est plus nécessaire d'adapter la base au principe actif. Contrairement à l'état de l'art actuel, il n'est plus indispensable et contraignant de choisir la base support en fonction de la nature du principe actif. En effet, les compositions cosmétiques disponibles sur le marché procèdent toujours par des assemblages uniques sans aucune possibilité d'intervention ou d'amélioration.

Ainsi, un principe actif solide, par exemple la vitamine C, devait être incorporé dans une base permettant sa dissolution et selon des pourcentages limités. Un principe actif hydrophobe ou hydrophile devait être mis en œuvre avec une base compatible.

L'invention apporte en outre un avantage important sur le plan économique car il n'est plus nécessaire de préparer, stocker ou modifier les bases actuellement disponibles sur le marché en vue de préparer une composition déterminée. La mise à disposition du praticien d'une base universelle unique élimine le recours à une panoplie de bases dont il fallait assurer la conservation et veiller à la durée d'utilisation.

Par huile riche en acides gras essentiels polyinsaturés et plus particulièrement en oméga-3, on entend une huile qui présente un pourcentage massique élevé d'acide linolénique, c'est-à-dire au moins 15%, et un rapport massique oméga-6 sur oméga-3 relativement faible, c'est-à-dire inférieur ou égal à 5.

L'huile décrite ci-dessus permet non seulement de solubiliser certains principes actifs, mais a également une action bénéfique reconnue sur la peau en favorisant la restauration du film hydrolipidique et la production, dans certaines conditions, d'oméga-3 cutanés.

Dans le cadre de l'invention, l'huile présente dans la composition peut notamment être une des huiles suivantes : l'huile de cameline, connue aussi sous la dénomination huile de pépins de cameline, l'huile de graines de citrouille, l'huile de chanvre, huile de pépin de cassis l'huile de lin, huile d'argan, huile d'olive, huile d'onagre, huile de bourrache et l'huile de pérille. Toutes ces huiles présentent les caractéristiques précédemment décrites.

Dans le cadre de l'invention, l'eau présente dans la base universelle est de l'eau habituellement utilisée en cosmétique ou en dermatologie. Elle peut notamment être de l'eau distillée, de l'eau déminéralisée ou au contraire une eau minérale, une eau thermale, etc.

La glycérine, ou glycérol, de formule HOH₂C-CHOH-CH₂OH, est un polyol bien connu en cosmétique, permettant notamment de pallier certaines fonctions lipides défaillantes de la peau. La glycérine permet entre autres une meilleure hydratation, la restauration des défenses naturelles, la régulation de la desquamation, l'amélioration de l'élasticité de la peau et la réparation de l'épiderme.

Par autre alcool, on entend tout alcool, à l'exception de la glycérine, utilisé couramment en cosmétique et/ou en dermatologie, tel que l'éthanol, propanol, propanediol, propan-2-ol, propylène glycol, méthylène glycol, triméthylène glycol, etc.

Par agents émulsifiants, on entend tous composés favorisant l'émulsion, tels que les tensioactifs, les antiagglomérants, les stabilisateurs, etc. On peut citer notamment (noms INCI UE) : sodium acrylate/sodium acryloyldimethyl taurate copolymer, polysorbate 80 et xanthan gum. On peut également introduire ces trois composés ensemble dans la base universelle.

La base universelle peut être par exemple un gel ou une émulsion complexe (émulsion triple, nanoémulsion, microencapsulation, microfluide etc.).

Dans le cadre de l'invention, les peptides biomimétiques utilisés sont de formule générale A-X-Gly-His-Lys-Y. A représente un atome d'hydrogène ou un radical d'un acide monocarboxylique de formule générale R-COOH dans laquelle R représente un radical aliphatique comprenant un à onze atomes de carbone. X représente une liaison simple ou la lysine, l'arginine ou l'ornithine. Y représente un groupe -OH ou NH2. Enfin, Gly, His et Lys représentent respectivement la glycine, l'histidine et la lysine. Les peptides sont typiquement présents dans la base universelle suivant des proportions de 1 à 20 ppm, préférentiellement de 3 à 10 ppm.

Un des peptides préféré est celui correspondant à la formule : H-Gly-His-Lys-OH.

On peut éventuellement combiner dans la base universelle plusieurs peptides, tels que ceux commercialisés par des sociétés telles que par exemple Lucas Meyer, Unipex, Mibelle, Caregen, Sederma. Les dénominations commerciales des peptides commercialisés par ces sociétés sont par exemple Kollaren®, Thymulen®, Melitane®, ECM Protect®, B-White, etc. Ceci permet de couvrir une très large gamme de besoins cutanés.

Les peptides biomimétiques sont des principes actifs puissants utilisés en cosmétique et en dermatologie. Ils stimulent de façon précise certaines fonctions cutanées défaillantes. Ainsi, la base universelle est en elle-même active cosmétiquement et/ou dermatologiquement. Ainsi, il est possible de préparer différentes bases universelles comprenant chacune un ou plusieurs peptides biomimétiques sans perturber la stabilité de la base universelle.

Optionnellement, la base universelle peut en outre comprendre de l'acide hyaluronique, pour son effet lubrifiant et hydratant, suivant une concentration de préférence inférieure à 5% en masse.

La base universelle présente en elle-même une activité cutanée grâce entre autres aux peptides biomimétiques. Elle peut donc cibler diverses pathologies et dysfonctionnements cutanés. On peut ainsi préparer une base universelle pour toutes les pathologies, dysfonctionnements ou besoins cutanés rencontrés par les professionnels du domaine, puis renforcer, potentialiser ou compléter leurs effets avec différents principes actifs, notamment ceux qui ont été cités ci-dessus.

La base universelle permet notamment de solubiliser un très grand nombre de principes actifs et de familles de principes actifs, par exemple solides comme la vitamine C.

Parmi les principes actifs utilisables, on peut notamment citer :
- l'acide férulique,
- la phlorétine,
- la SOD ou Super Oxyde Dismutase,
- le rétinol et/ou autres rétinoïdes,
- le niacinamide ou Vitamine B3,
- le DMAE ou diméthyle aminoéthanol,
- le resvératrol,
- les isofalvones riches en la génistéine et daidzéine,
- l'acide phytique,
- l'acide kojique,
- l'acide glycolique,
- l'arbutine,
- l'acide azélaïque,
- la N-acétyl cystéine,
- la tyrosine,
- l'acide alpha-lipoïque,
- la vitamine C,
- la vitamine B5,
- les sels de zinc et de magnésium,
- l'acide salicylique,
- des extraits végétaux riches en rutine,
- l'aloe vera,
- les madécassosides,
- la caféine,
- le silicium organique,
- la L-Carnitine,
- les lactopeptides et/ou bacteriocines,
- l'urée,
- l'acide lactique,
- l'acide mandélique,
- l'acide ursolique,
- la resorcinole,
- les céramides
- l'acide hyaluronique
- l'hydroquinone et ses dérivés,
et /ou autres extraits végétaux riches en flavonoïdes qui peuvent avoir un effet bénéfique sur la peau.

Les principes actifs utilisés dans l'invention présentent un degré de pureté élevée et peuvent être pulvérulents ou liquides. On peut optionnellement les disperser dans des produits galéniques tels que des excipients antiagglomérants, fluidifiants, etc....

La préparation des compositions cosmétiques et/ou dermatologiques de manière extemporanée peut être réalisée de façon simple en prévoyant un kit, par exemple constitué d'un bouchon et d'un flacon, les deux conditionnements étant étanches avant utilisation.

Le bouchon contient un ou plusieurs principes actifs. Le flacon contient la base universelle.

Le bouchon est apte à être vissé sur le flacon. Lorsqu'on visse le bouchon sur le flacon, il suffit d'exercer une pression sur le haut du bouchon pour libérer par rupture d'une membrane le principe actif et le verser dans la base universelle. La solubilisation du principe actif dans la base universelle se fait par simple agitation, optionnellement avec un appareil adapté.

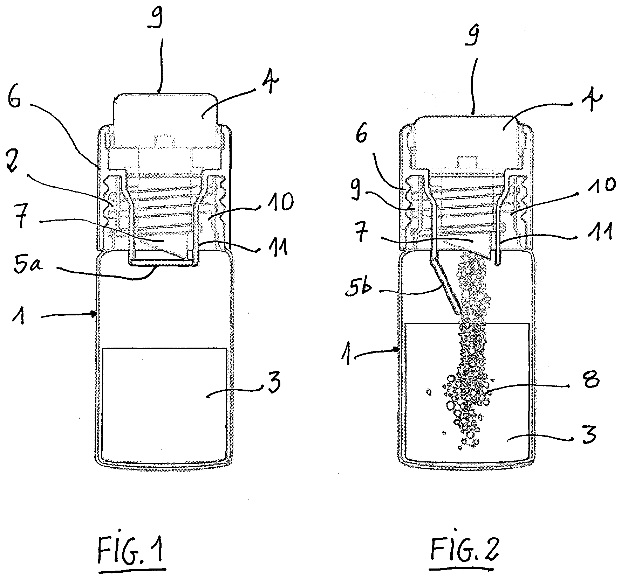

La figure 1 représente un flacon 1 contenant la base universelle 3 et une partie supérieure 10 munie d'un taraudage 2 sur ses bords extérieurs, ce flacon 1 étant vissé à un bouchon 4 creux contenant le principe actif 8. Le bouchon 4 présente un taraudage 6 intérieur complémentaire du taraudage 2 du flacon 1 et une membrane 5a dans une position fermée, garantissant l'étanchéité du bouchon.

Lorsqu'on appuie sur la partie supérieure 9 du bouchon 4, le principe actif 8 est versé dans le flacon 8 et donc se mélange à la base universelle 3.

La figure 2 représente le même flacon 1 vissé avec un bouchon 4 que dans la figure 1 mais après appui sur la partie supérieure 9 du bouchon 4. La membrane 5b est alors rompu par un élément 7 de la partie inférieure 11 du bouchon, ce qui permet au principe actif 8 de se déverser dans le flacon 1 et se mélanger à la base universelle 3.

On peut ajouter plusieurs doses unitaires du même principe actif pour augmenter l'effet de la composition cosmétique et/ou dermatologique préparée de manière extemporanée, ou bien choisir plusieurs principes actifs complémentaires, ou bien encore choisir des principes actifs ayant différents effets. Plusieurs dizaines de bouchons prédéterminés contenant chacun un ou plusieurs principes actifs à une dose définie peuvent ainsi être mis à disposition du pharmacien ou du médecin.

Afin que les quantités de principes actifs introduites dans le bouchon soient homogènes, les actifs purs peuvent être dispersés au sein d'un ingrédient pulvérulent tel que de l'amidon, du dextran, l'aérosil etc. Cet ingrédient peut éventuellement être liquide. Les principes actifs se présentent dans le bouchon sous leur forme la plus pure et suivant une concentration optimale. La concentration varie en fonction du principe actif et elle est conforme à la législation en vigueur.

L'assistance d'un professionnel tel qu'un pharmacien, un médecin esthétique ou un dermatologue peut s'avérer nécessaire. Cette assistance est en effet recommandée pour effectuer précisément le diagnostic de la peau, puis le choix et le dosage d'un ou de plusieurs principes actifs, l'homogénéisation du mélange et le conseil d'utilisation au consommateur (ex. : posologie). Le professionnel peut optionnellement effectuer le diagnostic de la peau du patient à l'aide d'un des appareils suivants : Cornéomètre®, Cutomètre®, Mobile Skin Scope MSS, Quantirides®, Sébumètre®, Mexamètre®, Dermalab® etc.

Toutes les concentrations exprimées en pourcentage dans la présente demande sont en pourcentage massique.

Dans le cadre général de l'invention, on peut notamment traiter les dysfonctionnement cutanés suivants : acné, acné rosacée, peaux grasses séborrhéiques, dermite séborrhéiques, peaux sensibles et/ou sensibilisées suite acte mécanique type dermabrasion, brulure N2 liquide et laser et/ou suite acte invasive type injections acide hyaluronique/mésothérapie rougeurs, prurit, psoriasis, cicatrisation, vieillissement accéléré et/ou chronologique, peeling, inflammation cutanée, effets des radiations UV, points noirs, excès de sébum, pores dilatés, taches pigmentaires, hyperpigmentation, irritations, couperose, dermatite atopique, infections bactériennes ou virales, perte de fermeté, rides et ridules, vergetures, cellulite, dermatoporose etc.

L'application sur la peau des préparations cosmétiques et/ou dermatologiques s'effectue de préférence au compte-goutte et/ou à la main.

On a réalisé de manière extemporanée un certain nombre de compositions cosmétiques et/ou dermatologiques répondant aux formulations suivantes. Les concentrations sont toujours massiques. Les pourcentages des peptides commerciaux dans la base universelle ne correspondent pas au pourcentage des peptides purs, car « peptides commerciaux » s'entend comme les formulations dans lesquelles sont vendus les différents peptides, la concentration finale du peptide pur étant toujours comprises entre 1 et 20 ppm.

### Exemple 1

Pour illustrer un exemple de mise en oeuvre de l'invention, on réalise une composition cosmétique contenant le peptide biomimétique Kollaren® pour traiter un effet dépigmentant, et on ajoute un bouchon contenant 390 mg, soit 3% de principe actif arbutine.

| **Base universelle** | **% en masse** |
|---|---|
| Huile de cameline | 12 |
| Éthanol | 24 |
| Glycérine | 2,5 |
| Emulsionnant | 3 |
| Peptide commercial : Kollaren® | 2 ppm |
| Eau | QSP |

| **Principe actif** | **% en masse** |
|---|---|
| Arbutine | 3 |

### Exemple 2

À un flacon contenant 13 ml de la composition selon l'exemple 1, on additionne, pour un désordre léger de la pigmentation d'un patient, deux principes actifs à l'aide d'un bouchon contenant 520 mg de Vitamine B3 (soit 4% de Vitamine B3 pour la composition totale) et de 3% en masse d'arbutine.

| **Base universelle** | **% en masse** |
|---|---|
| Huile de cameline | 12 |
| Éthanol | 24 |
| Glycérine | 2,5 |
| Emulsionnant | 3 |
| Peptide commercial Kollaren® | 3 ppm |
| Eau | QSP |

| **Principes actifs** | **% en masse** |
|---|---|
| Arbutine | 3 |
| Vitamine B3 | 4 |

### Exemple 3

À un flacon contenant la composition selon l'exemple 2 on additionne, pour un désordre important de type hyperpigmentation de la peau d'un patient, un principe actif supplémentaire à l'aide d'un bouchon contenant 3% d'arbutine. On ajoute donc deux fois de l'arbutine pour préparer la composition de manière extemporanée.

On obtient donc la composition suivante

| **Base universelle** | **% en masse** |
|---|---|
| Huile de cameline | 12 |
| Éthanol | 24 |
| Glycérine | 2,5 |
| Emulsionnant | 3 |
| Peptide commercial Kollaren® | 6 ppm |
| Eau | QSP |

| **Principes actifs** | **% en masse** |
|---|---|
| Arbutine | 6 |
| Vitamine B3 | 4 |

### Exemple 4

| **Base universelle** | **% en masse** |
|---|---|
| Huile de cameline | 10 |
| Éthanol | 20 |
| Glycérine | 2 |
| Emulsionnant | 1,75 |
| Peptides commerciaux | 11 ppm |
| Kollaren® GL 5000PPM | |
| Melitane® GL 1000PPM | |
| Thymulen® 47 GL 1000PPM, ECM Protect® GL 1000PPM | |
| Eau | QSP |

| **Principe actif** | **% en masse** |
|---|---|
| Vitamine C | 14 |
| SOD | 2 |

### Exemple 5

| **Base universelle** | **% en masse** |
|---|---|
| Huile de cameline | 8 |
| Éthanol | 12 |
| Glycérine | 1,2 |
| Emulsionnant | 4 |
| Peptides Kollaren® GL 5000PPM | 15 ppm |
| Eau | QSP |

| **Principe actif** | **% en masse** |
|---|---|
| Acide kojique | 2 |

### Exemple 6

| **Base universelle** | **% en masse** |
|---|---|
| Huile de cameline | 6 |
| Éthanol | 16 |
| Glycérine | 2 |
| Emulsionnant | 2 |
| Peptides Melitane® GL 1000PPM | 18 ppm |
| Acide hyaluronique | 0,3 |
| Eau | QSP |

| **Principe actif** | **% en masse** |
|---|---|
| Vitamine B5 | 5 |

### Exemple 7

| **Base universelle** | **% en masse** |
|---|---|
| Huile de cameline | 15 |
| Éthanol | 10 |
| Glycérine | 4 |
| Emulsionnant | 4,8 |
| Peptides Melitane® GL 1000PPM | 2 ppm |
| Eau | QSP |

| **Principe actif** | **% en masse** |
|---|---|
| Arbutine | 3 |

### Exemple 8

| **Base universelle** | **% en masse** |
|---|---|
| Huile de cameline | 23 |
| Éthanol | 28 |
| Glycérine | 9 |
| Emulsionnant | 2 |
| Peptides Melitane® GL 1000PPM | 5 ppm |
| Eau | QSP |

| **Principe actif** | **% en masse** |
|---|---|
| Arbutine | 6 |

### Exemple 9

| **Base universelle** | **% en masse** |
|---|---|
| Huile de cameline | 10 |
| Éthanol | 20 |
| Glycérine | 15 |
| Emulsionnant | 2 |
| Peptides Melitane® GL 1000PPM | 7 ppm |
| Eau | QSP |

| **Principe actif** | **% en masse** |
|---|---|
| Arbutine | 9 |

### Exemple 10

| **Base universelle** | **% en masse** |
|---|---|
| Huile de cameline | 10 |
| Éthanol | 20 |
| Glycérine | 15 |
| Emulsionnant | 2 |
| Peptides Melitane® GL 1000PPM | 2 ppm |
| Eau | QSP |

| **Principe actif** | **% en masse** |
|---|---|
| Arbutine | 3 |
| Aloe vera | 5,4 |
| Vitamine C | 7 |

### Exemple 11

| **Base universelle** | **% en masse** |
|---|---|
| Huile de cameline | 10 |
| Éthanol | 20 |
| Glycérine | 15 |
| Emulsionnant | 2 |
| Peptides Melitane® GL 1000PPM | 2 ppm |
| Eau | QSP |

| **Principe actif** | **% en masse** |
|---|---|
| Arbutine | 3 |
| Vitamine C | 7 |

### Exemple 12

| **Base universelle** | **% en masse** |
|---|---|
| Huile de cameline | 10 |
| Éthanol | 20 |
| Glycérine | 15 |
| Emulsionnant | 2 |
| Peptides Melitane® GL 1000PPM | 3 ppm |
| Eau | QSP |

| **Principe actif** | **% en masse** |
|---|---|
| Vitamine C | 7 |

### Exemple 13

| **Base universelle** | **% en masse** |
|---|---|
| Huile de cameline | 12 |
| Éthanol | 24 |
| Glycérine | 2,5 |
| Emulsionnant | 3 |
| Peptide commercial Kollaren® | 3 ppm |
| Eau | QSP |

| **Principes actifs** | **% en masse** |
|---|---|
| Arbutine | 6 |
| Vitamine B3 | 4 |
| Vitamine C | 7 |

### Exemple 14

| **Base universelle** | **% en masse** |
|---|---|
| Huile de cameline | 12 |
| Éthanol | 24 |
| Glycérine | 2,5 |
| Emulsionnant | 3 |
| Peptide commercial Kollaren® | 3 ppm |
| Eau | QSP |

| **Principes actifs** | **% en masse** |
|---|---|
| Vitamine B3 | 4 |
| Sulfate de zinc | 5 |

On a indiqué ci-dessus les constituants principaux de la base universelle, mais il va de soi que celle-ci est complétée par l'addition d'ingrédients habituels utilisés dans le domaine cosmétique en faible pourcentage. Ces ingrédients sont destinés à conférer certaines propriétés aux crèmes et gels préparés par la suite comme par exemple la texture. A titre d'exemple, on peut citer 0,6% de SYMDIOL® 68, 1,5% de SIMULGEL EG®, ou encore 0,25% de RHODICARES S®.

Dans les exemples précédents, on a cité l'arbutine, la vitamine B3, la Vitamine C, la SOD, l'acide kojique, l'aloe vera et la vitamine B5, mais il va sans dire que tous les principes actifs indiqués précédemment peuvent être utilisés selon les proportions indiquées avec les mêmes bases. Le choix d'un principe seul ou composé dépend de l'affection cutanée à traiter et de la gravité de cette affection. On pourra également combiner une application d'une préparation jour et une application d'une préparation nuit ou également des préparations pour le contour des yeux, les mains et le décolleté avec des proportions de principes actifs différents. Le prescripteur praticien aura toute latitude pour définir la composition à mettre en œuvre suivant le diagnostic qu'il établira.

On sait que l'arbutine inhibe la production de mélanine, pigment responsable de la coloration de la peau. Il éclaircit la peau, réduit l'intensité des taches et homogénéise le teint. Les essais effectués avec une composition selon l'exemple 1 montrent qu'après un traitement de 30 jours, on observe un éclaircissement net des taches pymentaires et après 60 jours de traitement une atténuation quasi complète de ces taches.

Ainsi, pour une affection liée aux UV (héliodermite), on peut prévoir une préparation constituée de 14% de vitamine C et de 2% de SOD (exemple 4) en application de jour et une préparation constitué de 6% d'arbutine, 4% de vitamine B3 et 7% de vitamine C (exemple 13) en application jour et nuit. On sait là encore que la vitamine C protège la peau des dommages occasionnés par les radicaux libres dus au stress environnemental (pollution, UV), stimule la synthèse du collagène et le renouvellement des fibroplastes, inhibe la tyrosinase, unifie le teint.

Là encore, les essais effectués avec la composition selon l'exemple 13 montrent au bout de 60 jours le rétablissement d'une peau nette et remodelée.

Enfin, les essais effectués avec la composition selon l'exemple 14 montrent après 7 jours de traitement un éclaircissement de la peau et une réduction des boutons d'acné.

Il en est de même de l'aloe vera qui aide à apaiser les peaux fragilisées et à réduire l'inflammation. Il possède une action enzymatique, légèrement exfoliante et astringente.

Les propriétés connues de ces trois principes actifs indiqués ci-dessus permettent de comprendre tout l'intérêt de l'invention de pouvoir combiner ces actifs à l'aide d'une base unique.

Les essais conduits à partir des compositions des exemples 1 à 14 ont donné d'excellents résultats dans le traitement d'affections variées de la peau ou pour répondre aux différents besoins cutanés. Ces compositions sont dotées d'une excellente stabilité et un stockage à température ambiante pendant 30 jours n'a conduit à aucune dégradation de ces compositions. De plus, aucune perte d'efficacité des compositions ainsi préparées n'a été constatée.

Les compositions des exemples 1 à 14 ont chacune été réalisées de la même façon en remplaçant l'huile de cameline par les huiles suivantes : l'huile de graines de citrouille, l'huile de chanvre, l'huile de lin et l'huile de pérille

De la même façon, chacune des compositions suivant les exemples 1 à 14 a été réalisée en remplaçant l'éthanol par les alcools suivants : l'éthanol, le propanol, le propanediol, le propan-2-ol, le propylène glycol, le méthylène glycol, ou le triméthylène glycol. Aucune modification notable des compositions préparées n'a été notée.

Les tests de stabilité, d'efficacité et de dégradation des compositions modifiées ont donné les mêmes résultats que ceux obtenus avec l'huile de cameline. Il en est de même lorsqu'on remplace l'éthanol par des autres alcools cités ci-dessus.

Ainsi, l'invention permet pour la première fois de réaliser n'importe quelle composition cosmétique et/ou dermatologique à partir de principes actifs en permettant leur dissolution et leur solubilisation dans l'huile, ou dans l'eau, ou dans l'alcool. Ainsi, le praticien dispose d'une base unique dans laquelle il réalise une composition précise suivant l'affection à traiter qu'il a diagnostiquée en utilisant soit un seul principe actif dosé à volonté suivant la gravité de l'affection, soit une combinaison de plusieurs principes actifs.

Cette composition est d'autant plus facile à réaliser que ce praticien dispose d'une palette de principes actifs prédosés à sa disposition qu'il adaptera très facilement.

Comme indiqué précédemment, la base universelle mise en œuvre dans l'invention présente déjà un effet bénéfique sur la peau contrairement à toutes les bases disponibles sur le marché qui n'ont généralement aucun effet.

On a donc analysé l'effet cicatrisant de cette préparation. Un modèle d'altération des capacités réparatrices de la peau a été réalisé par l'application d'un dermocorticoïde à la surface de l'épiderme afin de quantifier la stimulation du renouvellement cellulaire. Ce modèle expérimental est pertinent, car il se rapproche des effets secondaires observés par les dermatologues après application cutanée répétée de dermocorticoïdes avec comme conséquence un amincissement de la peau, fragilité et sensibilité aux infections. La stimulation du renouvellement cellulaire a été étudiée par quantification du potentiel mitotique des cellules basales.

L'effet hydratant et restructurant de la préparation a été analysé après réalisation d'un modèle d'altération de la barrière cutanée par le lauryl sulfate de sodium (LSS) responsable d'une perte insensible en eau accompagnée d'altération de l'expression de l'involucrine (protéine servant d'assise structurale aux protéines de l'enveloppe cornée et détectée dans le cytoplasme de la couche granuleuse et des assises supérieures du corps muqueux). L'évaluation de l'expression de l'involucrine permettra de quantifier la protection apportée par la crème au niveau de la couche cornée et la stimulation de son métabolisme au niveau des kératinocytes de la peau.

L'effet apaisant de la préparation a été analysé après réalisation sur la peau d'un modèle de brûlure expérimentale (application d'un gel agarose à 80°C). Son activité apaisante a été étudiée en analysant la limitation à la fois de la destruction de l'épiderme (correspondant à l'extension de la brûlure) et de la dilatation vasculaire (méthode d'analyse des vaisseaux : Branchet et coll, Skin Pharmacol Appl Skin Physiol 1999 ; 12 :211-220).

A cette fin, on a préparé une base contenant 64% d'eau, 20% d'alcool, 10% d'huile de caméline, 2% de glycérine, 1,5% de SIMULGEL® EG, 0,6% de SYMDIOL® 68, 0,001ppm de Kollaren® et divers additifs utilisés dans le domaine cosmétique dans des pourcentages inférieurs à 2% comme par exemple des émulsifiants du type sodium acrylate/sodium acryloyldimethyl taurate copolymer, polysorbate 80, xantham gum.

### MATERIEL ET METHODES

### 1) Evaluation de l'effet cicatrisant de la préparation

### a) Cultures d'organes, maintien en survie

Des fragments de peau de 8 donneurs différents ont été mis en culture pendant 4 jours avec application d'un dermocorticoïde de classe II (Diprosone® crème contenant la bétaméthasone à 0,05%) à J0 et J1 permettant d'obtenir une diminution de l'index mitotique.

La préparation a été appliquée tous les jours de J1 à J4.

### b) Mise en évidence immunohistochimique des cellules en mitose (Ki67).

L'analyse de l'effet cicatrisant a été évaluée par quantification des cellules en mitose. Les fragments de peaux ont été fixés dans le liquide de Bouin et inclus en paraffine. La prolifération épithéliale a été analysée par immunohistochimie à l'aide d'un anticorps anti-Ki67 (marqueur des cellules en phases M, S, G1 et G2 du cycle cellulaire) . L'immunodétection a été réalisée à l'aide d'une technique d'immunoperoxydase indirecte en 3 couches, amplifiée (kit CsA, DAKO) et révélée en AEC (3-amino-9-éthylcarbazole).

Un comptage du nombre de cellules marquées a été effectué et rapporté au nombre total de cellules basales pour calculer le % de cellules marquées.

### RESULTATS

- peau témoin : 12±6,2 %
- peau altérée par dermocorticoïde : 7,5±2,5 %
- peau altérée et traitée : 11,7±3,2 %

On constate une amélioration significative de la peau altérée. Dans le modèle expérimental d'altération par dermocorticoïde, on met en évidence une diminution de l'index mitotique proche de la significativité (p=0,075) avec un pourcentage de 7,5 versus 12% au niveau des peaux témoins.

Après traitement par la préparation, on constate une relance significative de cet index de prolifération avec un pourcentage de cellules marquées de 11,7% comparativement aux peaux altérées expérimentalement (p = 0,017).

### 2) Evaluation de l'effet hydratant et restructurant de la préparation

### a) Cultures d'organes, maintien en survie

Des fragments de peau de 8 donneurs différents ont été maintenus en survie pendant 4 jours avec 2 applications topiques de LSS à 1% (lauryl sulfate de sodium) à J0 et J1 permettant d'obtenir une altération de la barrière cutanée. La Crème Gel a été appliquée tous les jours de J1 à J4.

### b) Mise en évidence immunohistochimique de l'involucrine

Les fragments de peaux ont été fixés dans le liquide de Bouin et inclus en paraffine. L'immunodétection a été réalisée à l'aide d'une technique d'immunoperoxydase indirecte en 3 couches (kit ABC Peroxydase, Vector Laboratories) et révélée en AEC.

L'intensité du marquage immunohistochimique de la couche granuleuse et du corps muqueux a été évaluée par l'expression du marquage à l'aide d'un score histologique semi-quantitatif:

### RESULTATS (score intensité)

- peau témoin : 2,4±0,8 %,
- peau altérée par LSS : 1,4±1,2 %,
- peau altérée et traitée : 3±0,9 %.

On constate une différence statistiquement significative par rapport à la peau témoin, avec P<0,05.

### 3) Evaluation de l'effet apaisant de la préparation

score 0 : marquage négatif,
score 1 : intensité faible du marquage,
score 2 : intensité modérée du marquage,
score 3 : intensité importante du marquage,
score 4 : intensité très importante du marquage.

La topographie du marquage au niveau de l'épithélium a également été analysée en vérifiant si le marquage est présent au niveau de la couche granuleuse et du corps muqueux à l'aide d'un score histologique semi-quantitatif :
score 1 : couche granuleuse de l'épithélium,
score 2 : 1/3 supérieur de l'épithélium,
score 3 : 2/3 de l'épithélium,
score 4 : 3/4 de l'épithélium (tout l'épithélium sauf la couche basale).

### RESULTATS (score topographique)

- peau témoin : 2+-0,8 %
- peau altérée par LSS : 1,7±1,5
- peau altérée et traitée : 2,5±1

On met ainsi en évidence une diminution significative (p = 0,04) de l'expression du marquage de l'involucrine après altération par LSS : score d'intensité de 1,4 par rapport à la peau témoin (score de 2,4).

Après traitement par la préparation, on constate une augmentation significative de l'expression de l'involucrine : score de 3 versus 1,4 en comparaison avec la peau altérée (p=0,003) .

On met également en évidence une diminution (non significative) après LSS de la distribution de l'involucrine au sein de l'épithélium avec un score de 1,73 versus 2 pour la peau témoin. Une augmentation (également non significative) de sa distribution est observée après traitement par la préparation avec un score de 2,5.

### 4) Evaluation de l'effet apaisant de la préparation

### a) Cultures d'organes, maintien en survie

Des fragments de peau de 8 donneurs différents pendant 4 jours. Le modèle expérimental d'altération épithéliale a été obtenu par brûlure après application d'un gel d'agarose à 85°C à J0. Immédiatement après, la Crème Gel a été appliquée tous les jours jusqu'à J4

L'effet apaisant a été évalué à la fois par analyse de la limitation des altérations de l'épihélium et de la vasodilatation.

### b) Analyse histologique de l'épithélium

Les fragments de peaux sont fixés dans le liquide de Bouin et inclus en paraffine.

Après coloration par l'hémalun-éosine, le niveau d'altération de l'épiderme est évalué : clarification ou nécrose éosinophile du cytoplasme, noyaux picnotiques ou clarifiés. Ainsi, le nombre de cellules présentant des signes de nécrose importante ou de clarification légère du cytoplasme ou du noyau est noté, par champ analysé. Pour chaque peau, le % de cellules altérées a été calculé (10 champs au grossissement 40 sur 3 coupes différentes), en déduisant le % de cellules altérées par la mise en survie (bien que faible) observé sur les peaux témoins.

### c) Analyse de la vasodilatation et de l'œdème

Les fragments de peaux ont été fixés dans le liquide de Bouin et inclus en paraffine. Après coloration par l'hémalun-éosine, l'effet apaisant a été mesuré par quantification morphométrique de la surface occupée par les capillaires, du % de capillaires dilatés et par la quantité d'œdème dans le derme superficiel.

Après coloration par l'hémalun-éosine, la dilatation vasculaire sera évaluée par un comptage du nombre de vaisseaux dilatés sur l'ensemble de la coupe histologique (16 champs au grossissement 40). Ce nombre sera rapporté au nombre total de vaisseaux afin de calculer le pourcentage de vaisseaux dilatés. Par ailleurs, une analyse morphométrique de la surface (µm²) occupée par la lumière des vaisseaux sera réalisée afin de déterminer la surface moyenne (µm²) occupée dans le derme par les vaisseaux.

L'évaluation de l'œdème sera réalisée à l'aide de scores semi quantitatifs:
- pas d'œdème: 0 (score 0)
- œdème très léger: + (score 1)
- œdème modéré: ++ (score 2)
- œdème important: +++ (score 3)

Une étude comparative a été réalisée entre :
- peau témoin,
- peau altérée par brûlure
- peau altérée et traitée par la Crème Gel.

### d) Expression des résultats et analyse statistique

Une étude comparative des résultats entre les peaux traitées par la crème et les peaux non traitées (témoin) ou contrôles (modèle d'altération par dermocorticoïde, LSS ou brûlure) a été réalisée à partir des moyennes ± SD.

L'analyse statistique a été effectuée par le test de Student apparié avec un risque alpha de 5%.

### RESULTATS

Peau + brûlure : 24,1±25,1 %
Peau + brûlure + préparation : 15,8±11,6 %

On constate une augmentation des altérations de l'épiderme après brûlure avec un pourcentage de cellules altérées de 24,1%. Une diminution de ces altérations non significative après traitement par la crème a été mise en évidence avec un pourcentage de cellules altérées de 15,8 %.

### 5) Analyse de la vasodilatation

On a mis en évidence après brûlure une dilatation des capillaires du derme avec un pourcentage de vaisseaux dilatés augmenté à 99,3 versus 87,6 pour la peau témoin (p = 0, 004) et une surface moyenne de 270, 8 µm² versus 171,6 µm² pour la peau témoin (p = 4,6.10⁻⁵).

Le traitement par préparation a permis de limiter de façon significative la dilatation des capillaires avec une surface moyenne de 197,8 µm² versus 270, 8 µm² pour la peau contrôle (p = 0,003). Le pourcentage de vaisseaux dilatés est également diminué à 91,25% de façon significative (p = 0,02).

### 5) Analyse de l'œdème

On a observé une augmentation significative de l'œdème dermique après brûlure avec un score de 2,26 versus 1,73 pour la peau témoin (p = 0,036).

Une diminution non significative de l'œdème a été mise en évidence après traitement par la préparation avec obtention d'un score histologique de 1,88.

### CONCLUSIONS GENERALES

L'effet cicatrisant de la préparation a été mis en évidence par obtention d'une restauration statistiquement significative de l'index mitotique de l'épithélium après altération expérimentale de la peau.

Les effets des compositions comprenant le ou les principes actifs ont été évalués de la même manière.

L'effet hydratant et restructurant de la préparation a été visualisé et quantifié après réalisation d'un modèle d'altération de la barrière cutanée par le lauryl sulfate de sodium par mise en évidence d'une augmentation de l'expression de l'involucrine au niveau de l'épiderme. L'augmentation de cette expression témoigne de la protection apportée par la crème mais également de la stimulation du métabolisme de l'involucrine au niveau des cellules de la partie supérieure de l'épithélium.

L'effet apaisant de la préparation a été quantifié de façon significative après réalisation sur la peau d'un modèle de brûlure expérimentale par mise en évidence de la limitation de la dilatation des capillaires de la peau. La limitation de la destruction de l'épiderme (correspondant à l'extension de la brûlure) a été observée de façon plus modérée.

L'incorporation du principe actif (seul ou multiple) dans la base décrite et utilisée comme précédemment permet de sublimer et d'intensifier l'effet de ce principe actif. Bien entendu, l'action de la composition base universelle + principe actif permet une action plus rapide et bénéfique par rapport à une composition comprenant une base classique incorporant ce même principe actif.

## Revendications

1. Produit extemporané pour des soins cosmétiques et/ou dermatologiques comprenant au moins :
- un ou plusieurs principes actifs acceptables dans le domaine des soins cutanés cosmétiques et/ou dermatologiques, sous forme pulvérulente ou liquide, choisis grâce à un diagnostic personnalisé de la peau du patient d'une part ;
- et une base universelle d'autre part, comprenant :
- au moins une huile présentant une teneur massique en acide α-linolénique supérieure à 15% et un rapport massique oméga-6 sur oméga-3 inférieur ou égal à 5,
- de l'eau,
- de la glycérine,
- un ou plusieurs émulsifiants,
- au moins un autre alcool que la glycérine, acceptable dans les compositions cosmétiques et/ou dermatologiques,
- au moins un peptide biomimétique de formule générale A-X-Gly-His-Lys-Y dans laquelle :
- A représente un atome d'hydrogène ou un radical d'un acide monocarboxylique de formule générale R-COOH dans laquelle R représente un radical aliphatique comprenant un à onze atomes de carbone,
- X représente une liaison simple, la lysine, l'arginine ou l'ornithine,
- et Y représente un groupe -OH ou NH2,
ladite base universelle étant apte lors du mélange à renforcer, potentialiser, à compléter l'effet dudit ou desdits principes actifs et à solubiliser ledit ou lesdits principes actifs.

2. Produit extemporané selon la revendication 1, **caractérisé en ce que** l'huile est présente dans la base universelle suivant une concentration massique comprise entre 5 et 25%, et de préférence entre 7,5 et 15%.

3. Produit extemporané selon la revendication 1 ou la revendication 2, **caractérisé en ce que** l'huile est choisie parmi les huiles suivantes : huile de cameline, l'huile de graines de citrouille, l'huile de chanvre, l'huile de lin et l'huile de pérille.

4. Produit extemporané selon l'une des revendications précédentes, **caractérisé en ce que** la glycérine est présente dans la base universelle suivant une concentration massique comprise entre 1 et 20%, et de préférence entre 1 et 10%.

5. Produit extemporané selon l'une des revendications précédentes, **caractérisé en ce que** la base universelle comprend au moins un des émulsifiants dont le nom INCI UE est le suivant : sodium acrylate/sodium acryloyldimethyl taurate copolymer, polysorbate 80, xantham gum.

6. Produit extemporané selon l'une des revendications précédentes, **caractérisé en ce que** la base universelle comprend au moins un ou plusieurs émulsifiants suivant une concentration massique totale comprise entre 1 et 5%.

7. Produit extemporané selon l'une des revendications précédentes, **caractérisé en ce que** l'autre alcool est présent dans la base universelle suivant une concentration massique comprise entre 10 et 30%, et de préférence entre 15 et 25%.

8. Produit extemporané selon l'une des revendications précédentes, **caractérisé en ce que** l'autre alcool de la base universelle est choisi dans le groupe constitué par l'éthanol, le propanol, le propanediol, le propan-2-ol, le propylène glycol, le méthylène glycol, ou le triméthylène glycol.

9. Produit extemporané selon l'une des revendications précédentes, **caractérisé en ce que** le peptide biomimétique est présent dans la base universelle selon une concentration variant de 1 à 20 ppm, préférentiellement de 3 à 10 ppm.

10. Produit extemporané selon l'une des revendications précédentes, **caractérisé en ce que** la base universelle comprend au moins un peptide biomimétique de formule H-Gly-His-Lys-OH.

11. Produit extemporané selon l'une des revendications précédentes, **caractérisé en ce que** la base universelle comprend en outre de l'acide hyaluronique, préférentiellement suivant une concentration massique inférieure à 5%.

12. Produit extemporané selon l'une des revendications précédentes, **caractérisé en ce qu'**un ou plusieurs principes actifs sont choisis dans le groupe constitué par l'acide férulique, la phlorétine, la SOD ou Super Oxyde Dismutase, le rétinol et/ou autres rétinoides, le niacinamide et/ou Vitamine B3, le DMAE ou diméthyle aminoéthanol, le resvératrol, les isofalvones riches en la génistéine et daidzéine, l'acide phytique, l'acide kojique, l'acide glycolique, l'arbutine, l'acide azélaïque, la N-acétyl cystéine, la tyrosine, l'acide alpha-lipoïque, la vitamine C, la vitamine B5, les sels de zinc et de magnésium, l'acide salicylique, extraits végétaux riches en rutine, l'aloe vera, les madécassosides, la caféine, le silicium organique, la L-Carnitine, les lactopeptides et/ou bacteriocines, l'urée, l'acide lactique, l'acide mandélique, l'acide ursolique, la resorcinole, les ceramides, l'acide hyaluronique, l'hydroquinone et ses dérivés, et des extraits végétaux riches en flavonoïdes.

13. Produit extemporané selon l'une des revendications précédentes, **caractérisé en ce qu'**un ou plusieurs principes actifs sont choisis parmi les familles du groupe constitué par les flavonoïdes, les chalcones, les sels de zinc, de calcium et de magnésium de l'acide gluconique, les vitamines et les acides alpha et bêta-hydroxylés.

14. Kit de préparation extemporanée d'une composition cosmétique et/ou dermatologique mettant en œuvre un produit extemporané selon l'une des revendications précédentes, **caractérisé en ce qu'**il est constitué d'un premier conditionnement étanche contenant la base universelle, et un second conditionnement étanche contenant au moins un principe actif, les deux conditionnements étant aptes à être solidarisés de façon réversible et comprenant un moyen permettant de réunir leur contenu par une simple manipulation ou pression.

## Patentansprüche

1. Bedarfsmäßiges Produkt für kosmetische und/oder dermatologische Pflege, umfassend mindestens:
- einerseits einen oder mehrere auf dem Gebiet der kosmetischen und/oder dermatologischen Hautpflege akzeptable Wirkstoffe in Pulver- oder flüssiger Form, welche Dank einer personalisierten Diagnose der Haut des Patienten ausgewählt werden;
- und andererseits eine universelle Base, umfassend:
- mindestens ein Öl, welches einen Massengehalt an α-Linolensäure von größer als 15% und ein Massenverhältnis von Omega-β zu Omega-3 von kleiner als oder gleich 5 aufweist,
- Wasser,
- Glyzerin,
- ein oder mehrere Emulgatoren,
- mindestens einen anderen Alkohol als Glyzerin, welcher für die kosmetischen und/oder dermatologischen Zusammensetzungen akzeptabel ist,
- mindestens ein biomimetisches Peptid mit der allgemeinen Formel A-X-Gly-His-Lys-Y, in welcher:
- A ein Wasserstoffatom oder ein Radikal einer Monocarbonsäure mit der allgemeinen Formel R-COOH darstellt, in welcher R ein aliphatisches Radikal darstellt, welches ein bis elf Kohlenstoffatome umfasst,
- X eine einfache Verbindung, Lysin, Arginin oder Ornithin darstellt,
- und Y eine OH- oder NH2-Gruppe darstellt,
wobei die universelle Base bei der Mischung geeignet ist, die Wirkung des oder der genannten Wirkstoffe zu verstärken, potenzieren, zu ergänzen und den oder die genannten Wirkstoffe löslich zu machen.

2. Bedarfsmäßiges Produkt nach Anspruch 1, **dadurch gekennzeichnet, dass** das Öl in der universellen Base gemäß einer Massenkonzentration vorliegt, welche zwischen 5 und 25% und vorzugsweise zwischen 7,5 und 15% liegt.

3. Bedarfsmäßiges Produkt nach Anspruch 1 oder Anspruch 2, **dadurch gekennzeichnet, dass** das Öl aus den folgenden Ölen ausgewählt wird: Leindotteröl, Kürbiskernöl, Hanföl, Leinöl und Perillaöl.

4. Bedarfsmäßiges Produkt nach einem der vorhergehenden Ansprüche, **dadurch gekennzeichnet, dass** das Glyzerin in der universellen Base gemäß einer Massenkonzentration vorliegt, welche zwischen 1 und 20% und vorzugsweise zwischen 1 und 10% liegt.

5. Bedarfsmäßiges Produkt nach einem der vorhergehenden Ansprüche, **dadurch gekennzeichnet, dass** die universelle Base mindestens einen der Emulgatoren umfasst, deren EU INCI-Bezeichnung wie folgt lautet: Natriumacrylat/Natrium-Acryloyldimethyl-Taurat-Copolymer, Polysorbat 80, Xantham-Gummi.

6. Bedarfsmäßiges Produkt nach einem der vorhergehenden Ansprüche, **dadurch gekennzeichnet, dass** die universelle Base mindestens einen oder mehrere Emulgatoren gemäß einer Gesamtmassenkonzentration umfasst, welche zwischen 1 und 5% liegt.

7. Bedarfsmäßiges Produkt nach einem der vorhergehenden Ansprüche, **dadurch gekennzeichnet, dass** der andere Alkohol in der universellen Base gemäß einer Massenkonzentration vorliegt, welche zwischen 10 und 30% und vorzugsweise zwischen 15 und 25% liegt.

8. Bedarfsmäßiges Produkt nach einem der vorhergehenden Ansprüche, **dadurch gekennzeichnet, dass** der andere Alkohol der universellen Base aus der Gruppe ausgewählt wird, welche aus Ethanol, Propanol, Propandiol, Propan-2-ol, Propylenglykol, Methylenglykol oder Trimethylenglykol besteht.

9. Bedarfsmäßiges Produkt nach einem der vorhergehenden Ansprüche, **dadurch gekennzeichnet, dass** das biomimetische Peptid in der universellen Base gemäß einer Massenkonzentration vorliegt, welche zwischen 1 und 20 ppm, vorzugsweise zwischen 3 und 10 ppm, variiert.

10. Bedarfsmäßiges Produkt nach einem der vorhergehenden Ansprüche, **dadurch gekennzeichnet, dass** die universelle Base mindestens ein biomimetisches Peptid mit der Formel H-Gly-His-Lys-OH umfasst.

11. Bedarfsmäßiges Produkt nach einem der vorhergehenden Ansprüche, **dadurch gekennzeichnet, dass** die universelle Base darüber hinaus Hyaluronsäure, vorzugsweise gemäß einer Massenkonzentration von kleiner als 5%, umfasst.

12. Bedarfsmäßiges Produkt nach einem der vorhergehenden Ansprüchen, **dadurch gekennzeichnet, dass** ein oder mehrere Wirkstoffe aus der Gruppe ausgewählt werden, welche aus Ferulasäure, Phloretin, SOD oder Superoxid-Dismutase, Retinol und/oder andere Retinoide, Niacinamid und/oder Vitamin B3, DMAE oder Dimethylaminoethanol, Resveratrol, Isofalvonen, die reich an Genistein und Daidzein sind, Phytinsäure, Kojisäure, Glykolsäure, Arbutin, Azelainsäure, N-Acetyl-Cystein, Tyrosin, Alpha-Liponsäure, Vitamin C, Vitamin B5, Zink- und Magnesiumsalzen, Salicylsäure, Pflanzenextrakten, die reich an Rutin, Aloe Vera sind, Madecassosiden, Koffein, organischem Silizium, L-Carnitin, Milch-Peptiden und/oder Bakteriozinen, Harnstoff, Milchsäure, Mandelsäure, Ursolsäure, Resorcin, Ceramiden, Hyaluronsäure, Hydrochinon und dessen Derivaten und Pflanzenextrakten, die reich an Flavonoiden sind, besteht.

13. Bedarfsmäßiges Produkt nach einem der vorhergehenden Ansprüche, **dadurch gekennzeichnet, dass** ein oder mehrere Wirkstoffe aus den Familien der Gruppe ausgewählt werden, welche aus Flavonoiden, Chalkonen, Zink-, Kalzium- und Magnesiumsalzen der Gluconsäure, Vitaminen und Alpha- und Beta-Hydroxysäuren besteht.

14. Set zur bedarfsmäßigen Bereitstellung einer kosmetischen und/oder dermatologischen Zusammensetzung unter Verwendung eines bedarfsmäßigen Produkts nach einem der vorhergehenden Ansprüche, **dadurch gekennzeichnet, dass** es aus einer ersten, dichten, die universelle Base enthaltenden Verpackung und einer zweiten, dichten, mindestens einen Wirkstoff enthaltenden Verpackung besteht, wobei die beiden Verpackungen geeignet sind, reversibel verbunden zu werden, und ein Mittel umfassen, welches es ermöglicht, ihren Inhalt durch eine einfache Handhabung oder Druck zu vereinen.

## Claims

1. An extemporaneous product for cosmetic and/or dermatological treatment comprising, at least:
- one or several active substances acceptable in the field of cosmetic and/or dermatological skin care, in pulverulent or liquid form, selected by a personal diagnostic of the skin of the patient on a first part,
- and a universal base comprising:
- at least one oil having a mass content of α-linoleic acid greater than 15% and a weight ratio of omega-6 to omega-3 of less than or equal to 5,
- water,
- glycerine,
- one or several emulsifiers,
- at least one alcohol other than glycerine, acceptable in cosmetic and/or dermatological compositions,
- at least one biomimetic peptide of the general formula A-X-Gly-His-Lys-Y in which:
- A represents a hydrogen atom or a radical of a monocarboxylic acid of the general formula R-COOH in which R represents an aliphatic radical comprising one to eleven carbon atoms,
- X represents a single bond, lysine, arginine or ornithine,
- and Y represents a group -OH or NH2,
said universal base being able to after mixing to reinforce, potentialize, complete the effect of said active substance(s) and to solubilise said active substance or substances.

2. An extemporaneous product according to Claim 1, wherein the oil present in the universal base has a mass concentration of between 5 and 25%, and preferably between 7.5 and 20%.

3. An extemporaneous product according to Claim 1 or Claim 2, wherein the oil is selected from among the following: camelina oil, pumpkin seed oil, hemp oil, flax seed oil, and perilla oil.

4. An extemporaneous product according to one of the above Claims, wherein the glycerine present in the universal base has a mass concentration of between 1 and 20%, and preferably between 1 and 10%.

5. An extemporaneous product according to one of the above Claims, wherein the universal base comprises at least one of the emulsifiers whose EU INCI name is as follows: sodium acrylate / sodium acryloyldimethyl taurate copolymer, polysorbate 80, xantham gum.

6. An extemporaneous product according to one of the above Claims, wherein the universal base comprises one or several emulsifiers with a total mass concentration of between 1 and 5%.

7. An extemporaneous product according to one of the above Claims, wherein the other alcohol present in the universal base has a mass concentration of between 10 and 30%, and preferably between 15 and 25%.

8. An extemporaneous product according to one of the above Claims, wherein the other alcohol in the universal base is selected from the group formed by ethanol, propanol, propanediol, propan-2-ol, propylene glycol, methylene glycol or trimethylene glycol.

9. An extemporaneous product according to one of the above Claims, wherein the biomimetic peptide present in the universal base has a concentration varying from 1 to 20 ppm, and preferentially from 3 to 10 ppm.

10. An extemporaneous product according to one of the above Claims, wherein the universal base comprises at least one biomimetic peptide of the formula H-Gly-His-Lys-OH.

11. An extemporaneous product according to one of the above Claims, wherein the universal base additionally comprises hyaluronic acid, preferentially at a mass concentration of less than 5%.

12. An extemporaneous product according to one of the above Claims, wherein one or several active substances are selected from the group formed by ferulic acid, phloretin, SOD or superoxide dismutase, retinol and/or other retinoids, niacinamide or Vitamin B3, DMAE or dimethyl aminoethanol, resveratrol, isofalvones rich in genistein and daidzein, phytic acid, kojic acid, glycolic acid, arbutin, azelaic acid, N-acetyl cysteine, tyrosine , alpha-lipoic acid, vitamin C, vitamin B5, zinc and magnesium salts, salicylic acid, plant extracts rich in rutin, aloe vera, the madecassosides, caffeine, organic silicon, L-Carnitine, lactopeptides and/or bacteriocins, urea, lactic acid, mandelic acid, ursolic acid, resorcinol, ceramides, hyaluronic acid, hydroquinone and its derivatives, and/or other plant extracts rich in flavonoids.

13. An extemporaneous product according to one of the above Claims, wherein one or several active substances are selected from among the families of the group formed by flavonoids, chalcones, zinc, calcium and magnesium salts of gluconic acid, vitamins and alpha and beta-hydroxy acids.

14. A kit for the extemporaneous preparation of a cosmetic and/or dermatological composition implementing an extemporaneous product according to one of the above Claims, wherein it is constituted by a first sealed container containing the universal base, and a second sealed container containing at least one active substance, the two containers being able to be reversibly joined together and comprising means to combine their content by a simple manipulation or pressure.
